# EUROPÄISCHE PATENTSCHRIFT

(11) **EP 1 009 410 B1**
(45) Veröffentlichungstag und Bekanntmachung des Hinweises auf die Patenterteilung: **12.03.2003**
(21) Anmeldenummer: 98931817.5
(22) Anmeldetag: 15.07.1998
(51) Int. Cl.: A61K 31/60, A61K 9/00, A61K 9/12, A61K 47/08, A61K 47/10, A61K 47/22, A61K 47/26

(54) **VERFAHREN ZUM HERSTELLEN VON STABILEN ACETYLSALICYLSÄURE-LÖSUNGEN**
METHOD FOR PRODUCING STABLE ACETYLSALICYLIC ACID SOLUTIONS
PROCEDE POUR PREPARER DES SOLUTIONS STABLES D'ACIDE ACETYLSALICYLIQUE

(30) Priorität: 15.07.1997 AT 120697
(43) Veröffentlichungstag der Anmeldung: 21.06.2000
(73) Patentinhaber: Burghart, Walter, A-1030 Wien (AT); Burghart, Kurt, Dr., D-25548 Rosdorf (DE)
(72) Erfinder: Burghart, Walter, A-1030 Wien (AT); Burghart, Kurt, Dr., D-25548 Rosdorf (DE)
(74) Vertreter: Haffner, Thomas M., Dr.
(86) Internationale Anmeldenummer: AT9800173
(87) Internationale Veröffentlichungsnummer: WO99003474

(56) Entgegenhaltungen:
- EP-A- 0 055 635
- WO-A-92/07559
- WO-A-93/20815
- WO-A-97/27750
- FR-A- 2 258 865
- US-A- 3 316 150
- US-A- 4 228 162
- US-A- 5 723 453
- CHEMICAL ABSTRACTS, vol. 105, no. 18, 3. November 1986 Columbus, Ohio, US; abstract no. 158814, XP002080475 & CS 227 456 B (V.VALENTA ET AL.) 15. April 1986

## Beschreibung

Die Erfindung bezieht sich auf ein Verfahren zum Herstellen von stabilen Acetylsalicylsäure-Lösungen mit pharmazeutisch geeigneten, nicht wässrigen organischen Lösungsmitteln wie z.B. Dimethylisosorbid, Propylenglykol oder Diethylenglykolmonoethylether sowie auf stabile Lösungen von Acetylsalicylsäure und eine bevorzugte Verwendung dieser Lösungen zur schnellen dermalen oder sublingualen Wirkstoffresorption.

Die Instabilität von Acetylsalicylsäure in Arzneimittelzubereitungen ist schon seit langer Zeit bekannt. In Abhängigkeit von einer Reihe von Faktoren tritt relativ rasche Hydrolyse der Acetylsalicylsäure in Salicylsäure und Essigsäure ein. Neben dem bekannten Hydrolyseprodukt Salicylsäure sind auch Acetylsalicylsäureanhydrid und Acetylsalicylosalicylsäure als Verunreinigungen bekannt. Die Zersetzung von Aspirin führt zu einem weitestgehenden Verlust der pharmakologischen Wirksamkeit insbesondere bei der Indikation Herzinfarktprophylaxe und führt dazu, daß Acetylsalicylsäure in der Regel nur in Form von festen Zubereitungen auf den Markt gelangt. Feste Zubereitungen können wiederum in aller Regel nur oral verabreicht werden, wobei die rasche Zersetzung, insbesondere im sauren Milieu des Magens, bei der Resorption in der Magenschleimhaut, sowie durch den first pass in der Leber stattfindet, sodaß nur etwa die Hälfte der Acetylsalicylsäure in unzersetzter Form in die Blutbahn gelangt. Der während der Resorption entstehende Metabolit Salicylsäure wird für die Nebenwirkungen, wie z.B. Magenblutungen verantwortlich gemacht und die aufgrund des hohen Wirkstoffabbaus während der Resorption erforderliche Überdosierung führt zu einer bedeutenden Mehrbelastung des Organismus.

Neben der antiinflammatorischen, fiebersenkenden und analgetischen Wirkung von Aspirin wurde Acetylsalicylsäure auch als Antirheumatikum und zur Infarktprophylaxe eingesetzt, wobei eine Reihe von Versuchen bereits darauf abzielt, die Zersetzung der pharmazeutischen Zubereitung im Magen hintanzuhalten, um auf die Art und Weise unerwünschte Nebeneffekte zu vermeiden. Vor allem in der Dauertherapie ist es vorteilhaft die Konzentration von Salicylsäure im Organismus möglichst niedrig zu halten. Deshalb wurden stabile Acetylsalicylsäurelösungen entwickelt, die zudem geeignet sind bei dermaler Anwendung den Wirkstoff rasch in die Haut und weiter in die Blutbahn zu transportieren, damit dort eine systemische Wirkung ohne gastrointestinale Nebenwirkungen eintritt. Zur industriellen Vermarktung müssen diese Lösungen langzeitstabil sein.

Die Hydrolyse von Aspirin zu Salicylsäure und Essigsäure folgt einer Kinetik erster Ordnung und wird sowohl von Säuren als auch von Basen katalysiert. Aufgrund der raschen Hydrolyse von Aspirin in wässrigen Medien wurden bisher Versuche unternommen, flüssige Zubereitungen von Aspirin in nicht wässrigen Lösungsmitteln, wie beispielsweise Propylenglykol, Ethylalkohol, Glycerin oder Polyethylenglykol vorzunehmen. Spuren von Feuchtigkeit, Umesterungen od.dgl. können bei all diesen Lösungsmitteln jedoch nicht vermieden werden, sodaß auch hier nur eine unzureichende Stabilität erzielt wird. Bei der Verwendung von Polyethylenglykol und längerer Lagerung wurde auch eine Umesterung in Salicylsäure und acetyliertes Polyethylenglykol beobachtet. Aus diesem Grunde wurden bereits veresterte Polyethylenglykole in flüssigen Zubereitungen vorgeschlagen.

Eine flüssige Zubereitung unter Verwendung von Dimethylisosorbid ist der US-A 4 228 162 zu entnehmen. Eine derartige flüssige Lösung zeichnet sich zwar durch bedeutend bessere Stabilität als eine Reihe von anderen Lösungen aus. Für eine erfolgreiche Vermarktung und die geforderte Langzeitstabilität sind aber auch derartige Lösungen nicht ausreichend stabil. Zudem findet bei der Anwendung nur eine langsame dermale Resorption statt. Die Haut ist lange mit Flüssigkeit benetzt und es ist daher leicht möglich, daß die Lösung durch Kleidungsstücke aufgesaugt wird und der Wirkstoff nicht zur Aufnahme in den Körper zur Verfügung steht.

Aus der EP 55 635 A1 ist eine dermale Zubereitung von Acetylsalicylsäure bekanntgeworden, welche in Form eines Gels aufgebracht werden kann. Die dermale Applikation, welche besonders hohe Anforderungen an die Stabilität der Lösung stellt, hat hiebei den Vorteil, daß der unerwünschte First-Pass-Effekt vermieden werden kann und gastrointestinale Irritationen wie Magenblutungen unterbleiben. Voraussetzung für eine entsprechend wirksame Zubereitung ist aber ein hohes Maß an Stabilität, wobei in der EP 55 635 A1 unter anderem Diethylenglykolmonoethylether und Propylenglykol vorgeschlagen wurde. Die hier beschriebene Zubereitung verwendete ein Carboxyvinylpolymer zur Ausbildung eines Gels, wobei zur weiteren Stabilisierung unter anderem Ethylendiamin-tetraessigsäure (EDTA) eingesetzt wurde.

Auch diese bekannte Zubereitung erfüllt nicht die geforderte Langzeitstabilität und rasche Wirkstoffresorption. Die vorliegende Erfindung zielt daher darauf ab, ausgehend von einer Lösung der eingangs genannten Art die Stabilität und Resorption derartiger Lösungen wesentlich zu verbessern.

Zur Lösung dieser Aufgabe besteht das erfindungsgemäße Verfahren zum Herstellen von stabilen Acetylsalicylsäure-Lösungen mit pharmazeutisch unbedenklichen, nicht wässrigen organischen Lösungsmitteln im wesentlichen darin, daß der Lösung 0,005 - 2 Gew.% eines cyclischen Säureimides und/oder 0,005 - 2 Gew.% einer Sulfaminsäure sowie bevorzugt 1 - 40 Gew.% bezogen auf das Gewicht der Lösung einer Verbindung der allgemeinen Formel 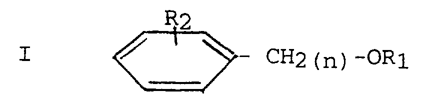 worin R₁ H, CH₃ oder C₂H₅, n = 0 oder 1 und R₂ H oder einen oder mehrere Niedrigalkyl-, Niedrigalkyloxy- oder Niedrigalkenylreste mit 1 bis 5 C-Atomen bedeutet, zugesetzt wird.

Die oben aufgeführten Verbindungen der allgemeinen Formel I sind oft Bestandteile von ätherischen Ölen oder pflanzlichen Extrakten. So enthält, z.B. Anisöl, mehr als 90 % Anethol oder Fenchelöl 50 bis 60 % Anethol, sodaß auch diese Öle oder Extrakte entsprechend ihrer Konzentration an oben aufgeführten Verbindungen eingesetzt werden können.

Überraschenderweise hat sich nun gezeigt, daß durch Verwendung der genannten Zusätze die Langzeitstabilität der Lösungen wesentlich verbessert werden kann, wobei die zulässige Lagerzeit um einen Faktor von wenigstens zwei erhöht werden konnte. Wesentlich hierbei ist, daß die freie Säure und nicht etwa Na-Salze, wie sie in Süßungsmitteln anzutreffen sind, eingesetzt werden, da die Na-Salze die Lösungen destabilisieren. Der Konzentrationsbereich, in welchem z.B. Saccharin als Süßstoff eingesetzt werden kann, liegt an der Untergrenze der für die Stabilisierung verwendeten Konzentrationen, wobei sich überraschenderweise gezeigt hat, daß Ethanol als Lösungsmittel nicht geeignet ist und ethanolische Lösungen des Wirkstoffes nicht mit Sacharin stabilisiert werden konnten.

Die dünnflüssigen Lösungen ziehen innerhalb von wenigen Minuten vollständig und irreversibel in die Haut ein, sodaß sich keine unerwünschten Einflüsse von außen, wie z.B.: Kleidungsstücke, Verteilung auf andere Gegenstände, ergeben und lassen sich wiederum ausgezeichnet mittels Dosierpumpe auf die exakten Acetylsalicylsäure-Gehalte dosieren. Neben Saccharin und/oder Cyclaminsäure haben sich überraschenderweise Verbindungen der allgemeinen Formel I, zu welchen bevorzugt Anethol oder Benzylalkohol zu zählen ist, als besonders vorteilhaft erwiesen. Der genaue Mechanismus, aufgrund dessen unter Verwendung der erfindungsgemäß vorgeschlagenen Stabilisatoren die Langzeitstabilität wesentlich verlängert wird, konnte nicht definitiv geklärt werden. Der Ersatz von Cyclaminsäure oder Saccharin durch andere organische Säuren wie Sorbinsäure, Benzoesäure oder Nicotinsäure ergab keine entsprechende Langzeitstabilität mehr. Die im Langzeittest jeweils festgestellten Werte für das in der Zeit gebildete Hydrolyseprodukt Salicylsäure lagen jedenfalls durchwegs weit unter der Hälfte der bei bekannten als stabil bezeichneten Lösungen beobachteten Werte.

Mit Vorteil wird das erfindungsgemäße Verfahren so durchgeführt, daß als Sulfaminsäure Cyclaminsäure eingesetzt wird. Cyclaminsäure hat ebenso wie Saccharin, welches bevorzugt als cyclisches Säureimid eingesetzt wird, in den genannten Konzentrationen zu wenigstens einer Verdoppelung der Haltbarkeit geführt.

Die Verwendung von Anethol und/oder Benzylalkohol führt insbesondere bei einer dermalen Anwendung zu besonderen Vorteilen, da derartige in der Lösung verwendete Zusätze nach der Applikation schnell wieder eine trockene Haut ergeben, und der Wirkstoff mit dem Lösungsmittel außerordentlich schnell resorbiert wird und damit die transkutane Permeabilität für Acetylsalicylsäure gesteigert werden kann. Um eine Entfettung der Haut bei der Anwendung zu vermeiden, kann der Rezeptur eine geringe Menge Ölsäure zugesetzt werden. Statt Ölsäure können auch andere Öle oder fettende Substanzen zugesetzt werden, wie z.B. Vitamin E (Acetat).

Eine besonders bevorzugte stabile Lösung von Acetylsalicylsäure ist hiebei dadurch gekennzeichnet, daß sie 5 - 15 Gew.% Acetylsalicylsäure, 10 - 40 Gew.% Propylenglykol, 0 - 40 Gew.% Dimethylisosorbid, 0 - 70 Gew.% Diethylenglykolmonoethylether und 0,005 bis 2 Gew.% Saccharin und/oder Cyclaminsäure, und/oder 1 bis 40 Gew.% Anethol enthält, wobei die Lösung bevorzugt 12,5 Gew.% Acetylsalicylsäure und je 30 Gew.% Propylenglykol und Dimethylisosorbid, die beide ganz oder teilweise durch Benzylalkohol oder Anethol, bzw. einer Mischung aus den beiden Bestandteilen ersetzt werden können, Rest Diethylenglykolmonoethylether sowie 0,1 bis 0,5 Gew.% bezogen auf die Lösung Saccharin und/oder Cyclaminsäure enthält.

Eine weitere besonders bevorzugte Lösung enthält neben Acetylsalicylsäure 20 Gew.% Propylenglykol, 65 Gew.% Diethylenglykolmonoethylether und 0,1 Gew.% Saccharin, Cyclaminsäure und/oder 0,5 bis 5 Gew.% Anethol. Teile von Diethylenglykolmonoethylether können hiebei durch Benzylalkohol oder Anethol oder Mischungen derselben ersetzt werden, wobei statt Saccharin 0,2 bis 0,5 Gew.% Cyclaminsäure bevorzugt eingesetzt werden können.

Eine weitere Verbesserung der Stabilität derartiger Lösungen läßt sich naturgemäß dadurch erzielen, daß die Lösungen unter weitestgehendem Luft- und Feuchtigkeitsabschluß aufbewahrt werden. Besonders bevorzugt liegt daher die erfindungsgemäße stabile Lösung als Pumpenspray mit Produktverschluß vor, wodurch sichergestellt wird, daß jeweils nur diejenige Menge Luft und damit Luftfeuchtigkeit zutreten kann, welche für das Verdrängen einer bestimmten dosierbaren Menge des Wirkstoffes in seiner Lösung erforderlich ist, und eine für pharmazeutische Zwecke notwendige exakte Wirkstoff-Dosierung ermöglicht wird.

Eine besonders bevorzugte stabile Lösung ist dadurch gekennzeichnet, daß sie 10 - 24 Gew.% Acetylsalicylsäure, 0,005 - 2 Gew.% Saccharin und/oder Cyclaminsäure, 20 - 80 Gew.% Methoxypropanol, insbesondere 1-Methoxy-2-propanol und ggf. 1 bis 30 Gew.% Benzylalkohol und/oder Anethol, sowie ggf. Propylenglykol und/oder Diethylenglykolmonoethylether und ggf. Ölsäure und Tocopherol (Vitamin E) enthält. Die Verwendung von 1-Methoxy-2-propanol als Lösungsmittel erlaubt eine wesentlich höhere Konzentration von Acetylsalicylsäure in Lösung. Wesentlich ist hier mit Rücksicht auf die rasche dermale Resorption die gelöste Menge und damit die Konzentration von Acteylsalicylsäure in Lösung, wobei bei Verwendung von 1-Methoxy-2-propanol auf höhere Mengen anderer Lösungsmittel oder Lösungsvermittler verzichtet werden kann und einem Austrocknen der Haut durch Zusatz von Ölsäuren und ggf. Vitamin E entgegengewirkt werden kann.

Aufgrund der hohen Langzeitstabilität eignet sich die erfindungsgemäße stabile Lösung insbesondere auch als Pumpenspray bevorzugt zur pharmazeutischen dermalen oder sublingualen Applikation.

Die Erfindung wird nachfolgend anhand von Ausführungsbeispielen im Vergleich zum Stand der Technik näher erläutert.

### Beispiel 1

Entsprechend dem Stand der Technik wurde 1 g Acetylsalicylsäure in 7 g Diethylenglykolmonoethylether (Transcutol) und 2 g Propylenglykol gelöst. Die Stabilität der Lösung wurde durch Bestimmung des Hydrolyseproduktes Salicylsäure verfolgt, wobei sich eine durchschnittliche Bildung von 0,076 Gew.% Salicylsäure pro Tag ergab (Lagerung bei 25° C). Ein Resorptionsversuch mit dem in der EP 55 635 A1 beschriebenen Gel bei transdermaler Applikation ergab relativ ungünstige Werte, da nach 60 min erst etwa die Hälfte des aufgetragenen Wirkstoffes resorbiert wurde.

### Beispiel 2

Der Lösung nach Beispiel 1 wurde 0,005 g Saccharin zugesetzt. Die in gleicher Weise bestimmte Menge des über die Zeit gebildeten Hydrolyseproduktes Salicylsäure ergab eine durchschnittliche Bildung von 0,0377 Gew.% pro Tag, woraus sich eine Verdoppelung der Haltbarkeit und entsprechende Verlängerung der Stabilität ergibt.

### Beispiel 3

Der Lösung nach Beispiel 1 wurden 0,01 g Saccharin zugesetzt. Die im Schnitt pro Tag gebildete Salicylsäuremenge sank hiebei auf 0,031 Gew.% ab, woraus eine weitere Erhöhung der Stabilität resultiert.

### Beispiel 4

Die Menge an Saccharin wurde ausgehend von der Lösung nach Beispiel 1 auf 0,05 g erhöht. Die über die Zeit gemittelte Zunahme des Hydrolyseproduktes wurde mit 0,025 Gew.% pro Tag gemittelt.

### Beispiel 5

Ausgehend von der Lösung nach Beispiel 1 wurde Saccharin in einer Menge von 0,1 g zugesetzt. Die im Mittel gebildete Menge an Salicylsäure pro Tag sank auf 0,0247 Gew.%.

### Beispiel 6

Zu Vergleichszwecken wurde eine Lösung aus 1 g Acetylsalicylsäure, 3 g Diethylenglykolmonoethylether, 3 g Dimethylisosorbid und 3 g Propylenglykol hergestellt. Die bei einer Lagertemperatur von 25° C über die Zeit bestimmte Bildung von Hydrolyseprodukten wurde mit 0,046 Gew.% pro Tag bestimmt.

### Beispiel 7

Ausgehend von einer Lösung nach Beispiel 6 wurden 0,02 g Cyclaminsäure zugesetzt. Die durchschnittliche Bildung von Salicylsäure sank auf einen Wert von 0,024 Gew.% pro Tag, woraus sich wiederum eine Verdoppelung der Haltbarkeit ergab.

### Beispiel 8

Ausgehend von einer Lösung nach Beispiel 6 wurden 0,1 g Cyclaminsäure zugesetzt. Die pro Tag gebildete Menge von Salicylsäure stieg auf 0,032 Gew.% an. Dieser Wert war aber immer noch deutlich besser als der Wert ohne Zusatz von Cyclaminsäure.

### Beispiel 9

Es wurde eine stabile Lösung aus 1 g Acetylsalicylsäure, 3 g Dimethylisosorbid, 3 g Diethylenglykolmonoethylether, 3 g Propylenglykol und 0,2 g Saccharin hergestellt. Die täglich gebildete Menge an Salicylsäure sank auf 0,0183 Gew.% ab, wodurch eine besonders stabile Lösung hergestellt werden konnte.

Die Resorptionsversuche bei dermaler Applikation ergaben, daß bereits nach 30 min mehr als die Hälfte des aufgetragenen Wirkstoffes resorbiert wurde.

### Beispiel 10

Es wurde eine stabile Lösung aus 1 g Acetylsalicylsäure, 3 g Benzylalkohol, 3 g Diethylenglykolmonoethylether, 3 g Propylenglykol und 0,05 g Saccharin hergestellt.

Mit dieser Lösung wurde ein Resorptionsversuch durchgeführt, wobei innerhalb von 15 min mehr als die Hälfte (57 %) und innerhalb von 30 min nahezu drei Viertel (73 %) des Wirkstoffes dermal resorbiert wurden.

### Beispiel 11

Es wurde eine stabile Lösung aus 1 g Acetylsalicylsäure, 3 g Anethol, 3 g Diethylenglykolmonoethylether, 3 g Propylenglykol und 0,05 g Saccharin hergestellt.

Die Resorptionsversuche bei dermaler Applikation ergaben nach 15 min 85 % und nach 30 min 93 % Resorption des Wirkstoffes.

### Beispiel 12

Es wurden nachfolgende Lösungen hergestellt:

| Menge einzelner Sprühgaben in mg | (A) **mg** | (B) **mg** | (C) **mg** | (D) **mg** | (E) **mg** |
|---|---|---|---|---|---|
| Acetylsalicylsäure ASS | 25,0 | 25,0 | 30,0 | 35,0 | 35,0 |
| Diethylenglykolmonoethylether | 90,0 | -- | 58,5 | 33,4 | 33,4 |
| 1-Methoxy-2-propanol | -- | 90,0 | 58,5 | 103,6 | 133,6 |
| Anethol | 20,0 | 20,0 | 40,0 | 20,0 | -- |
| Benzylalkohol | 20,0 | 20,0 | 10,0 | 10,0 | -- |
| Propylenglykol | 50,0 | 50,0 | 5,0 | 5,0 | 5,0 |
| Ölsäure | -- | -- | 2,0 | 2,0 | 2,0 |
| Saccharin | 1,0 | 1,0 | 1,0 | 1,0 | -- |
| | 205,1 | 205,1 | 204,1 | 210,0 | 210,0 |

In der Lösung (B) wurde Diethylenglykolmonoethylether gänzlich durch 1-Methoxy-2-propanol ersetzt. Diese Lösung zeigte unter gleichen Lagerbedingungen eine um den Faktor 1,75 bessere Stabilität als Lösung (A).

Von der Lösung (C) wurde ein Hautverträglichkeitstest am Menschen durchgeführt. Nach großflächiger Anwendung am Menschen über einen Zeitraum von 1 Monat und einer Dosierung von 150 mg wurden keine Irritationen festgestellt.

Die Lösung (D) stellt eine praxisgerechte, hochkonzentrierte ASS-Lösung dar, da sie in einer Sprühgabe von 210 mg 35 mg ASS dosiert.

Aus den Rezepturen (D) und (E) wurden nach 10 Minuten ungefähr 48 % resorbiert.

Nach 15 Minuten wurden aus der Lösung (D) 63 %, aus der Lösung (E) 55 % resorbiert. Diese 55 % Resorption nach 15 Minuten ist weitaus höher als vergleichend in der Literatur angegebene Lösungen und entspricht dem Resorptionverhalten der Lösung in Beispiel Nr. 10, in dem Verbindungen der allgemeinen Fomel (I) enthalten sind.

Vergleichsversuche mit 0,01 g und 0,05 g Benzoesäure ergaben im Vergleich zu den erfindungsgemäßen Stabilisatoren wesentlich höhere Werte für die Salicylsäurebildung. Ähnliche Werte wurden mit 0,05 g Sorbinsäure erzielt. Der Zusatz von 0,01 Nicotinsäure ergab schlechtere Werte als dem Stand der Technik entsprechen (0,74 Gew.% Salicylsäure/Tag), wobei die Erhöhung der Nicotinsäuremenge auf 0,05 g zu einer erheblichen Verschlechterung der Stabilität (2,05 Gew.% Salicylsäure/Tag) führte.

Vergleichende Resorptionsversuche ohne Zusatz der erfindungsgemäß vorgeschlagenen Substanzen ergaben durchwegs schlechtere Werte. Die Lösung in Dimethylisosorbid gemäß der US-A-4 228 162 führte nach 60 min zu einer Resorption von lediglich 21 Gew.% des Wirkstoffes. Auch Propylenglykol oder Transcutol als alleinige Lösungsmittel ergaben keine Resorption, welche mit den gemessenen Werten unter Einsatz der erfindungsgemäßen Zusätze vergleichbar waren.

Anethol und Benzylalkohol zeigten neben einer Verbesserung der Resorption auch eine deutlich wirkstoffstabilisierende Wirkung und können daher als teilweiser Ersatz der organischen Lösungsmittel gemeinsam mit Saccharin oder Cyclaminsäure als Stabilisatoren eingesetzt werden.

## Patentansprüche

1. Verfahren zum Herstellen von stabilen Acetylsalicylsäure-Lösungen mit nichtwässrigen pharmazeutisch verträglichen organischen Lösungsmitteln wie Dimethylisosorbid, Propylenglykol oder Diethylenglykolmonoethylether, **dadurch gekennzeichnet, daß** der Lösung 0,005 - 2 Gew.% eines cyclischen Säureimides und/oder 0,005 - 2 Gew.% einer Sulfaminsäure sowie bevorzugt 1 - 40 Gew.% bezogen auf das Gewicht der Lösung einer Verbindung der allgemeinen Formel 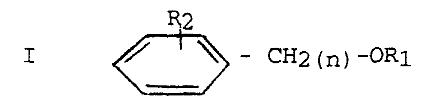 worin R₁ H, CH₃ oder C₂H₅, n = 0 oder 1 und R₂ H oder einen oder mehrere Niedrigalkyl-, Niedrigalkyloxy- oder Niedrigalkenylreste mit 1 bis 5 C-Atomen bedeutet, zugesetzt wird.

2. Verfahren nach Anspruch 1, **dadurch gekennzeichnet, daß** als Sulfaminsäure Cyclaminsäure eingesetzt wird.

3. Verfahren nach Anspruch 1 oder 2, **dadurch gekennzeichnet, daß** als cyclisches Säureimid Sulfonsäureimid bzw. Saccharin eingesetzt wird.

4. Verfahren nach Anspruch 1, 2 oder 3, **dadurch gekennzeichnet, daß** als Verbindung der allgemeinen Formel I Anethol und/oder Benzylalkohol eingesetzt wird.

5. Stabile Lösung von Acetylsalicylsäure nach einem der Ansprüche 1 bis 4, **dadurch gekennzeichnet, daß** sie 5 - 15 Gew.% Acetylsalicylsäure, 10 - 40 Gew.% Propylenglykol, 0 - 40 Gew.% Dimethylisosorbid, 0 - 70 Gew.% Diethylenglykolmonoethylether und 0,005 bis 2 Gew.% Saccharin und/oder Cyclaminsäure und/oder 1 bis 40 Gew.% Anethol und/oder Benzylalkohol enthält.

6. Stabile Lösung nach Anspruch 5, **dadurch gekennzeichnet, daß** die Lösung 12,5 Gew.% Acetylsalicylsäure und je 30 Gew.% Propylenglykol und Dimethylisosorbid, die beide ganz oder teilweise durch Benzylalkohol oder Anethol, bzw. einer Mischung aus den beiden Bestandteilen ersetzt werden können, Rest Diethylenglykolmonoethylether sowie 0,1 bis 0,5 Gew.% bezogen auf die Lösung Saccharin und/oder Cyclaminsäure enthält.

7. Stabile Lösung nach Anspruch 5 oder 6, **dadurch gekennzeichnet, daß** die Lösung 20 Gew.% Propylenglykol, 65 Gew.% Diethylenglykolmonoethylether und 0,1 Gew.% Saccharin und/oder Cyclaminsäure und/oder 0,5 - 5 Gew.% Anethol enthält.

8. Stabile Lösung nach einem der Ansprüche 5 bis 7, **dadurch gekennzeichnet, daß** die Lösung als Pumpenspray mit Produktverschluß vorliegt.

9. Stabile Lösung nach einem der Ansprüche 5 bis 8, **dadurch gekennzeichnet, daß** sie 10 - 24 Gew.% Acetylsalicylsäure, 0,005 - 2 Gew.% Saccharin und/oder Cyclaminsäure, 20 - 80 Gew.% Methoxypropanol, insbesondere 1-Methoxy-2-propanol und 1 - 30 Gew.% Benzylalkohol und/oder Anethol sowie Propylenglykol und/oder Diethylenglykolmonoethylether und Ölsäure und Tocopherol (Vitamin E) enthält.

10. Stabile Acetylsalicylsäure-Lösung nach einem der Ansprüche 5 bis 9 zur dermalen oder sublingualen Applikation.

## Claims

1. A method for producing stable acetylsalicylic acid solutions with nonaqueous pharmaceutically compatible organic solvents such as dimethyl isosorbide, propylene glycol or diethylene glycol monoethyl ether, **characterized in that** 0.005-2% by weight of a cyclic acid imide and/or 0.005-2% by weight of a sulfamic acid as well as, preferably, 1-40% by weight, based on the weight of the solution, of a compound of the general formula 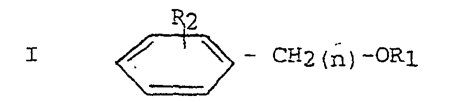 wherein R₁ represents H, CH₃ or C₂H₅, n = 0 or 1, and R₂ represents H or one or several lower alkyl, lower alkyloxy or lower alkenyl residues having 1 to 5 carbon atoms, is added to said solutions.

2. A method according to claim 1, **characterized in that** cyclamic acid is used as said sulfamic acid.

3. A method according to claim 1 or 2, **characterized in that** sulfonic acid imide or saccharine is used as said cyclic acid imide.

4. A method according to claim 1, 2 or 3, **characterized in that** anethol and/or benzyl alcohol is used as said compound of the general formula I.

5. A stable solution of acetylsalicylic acid according to any one of claims 1 to 4, **characterized in that** it contains 5-15% by weight of acetylsalicylic acid, 10-40% by weight of propylene glycol, 0-40% by weight of dimethyl isosorbide, 0-70% by weight of diethylene glycol monoethyl ether and 0,005 to 2% by weight of saccharin and/or cyclamic acid and/or 1 to 40% by weight of anethol and/or benzyl alcohol.

6. A stable solution according to claim 5, **characterized in that** said solution contains 12.5% by weight of acetylsalicylic acid and 30% by weight each of propylene glycol and dimethyl isosorbide, which may both be wholly or partially replaced with benzyl alcohol or anethol or a mixture of the two components, the balance being diethylene glycol monoethyl ether as well as 0.1 to 0.5% by weight, based on said solution, of saccharin and/or cyclamic acid.

7. A stable solution according to claim 5 or 6, **characterized in that** said solution contains 20% by weight of propylene glycol, 65% by weight of diethylene glycol monoethyl ether and 0.1% by weight of saccharin and/or cyclamic acid and/or 0.5-5% by weight of anethol.

8. A stable solution according to any one of claims 5 to 7, **characterized in that** said solution is present as a pump spray with a product closure.

9. A stable solution according to any one of claims 5 to 8 **characterized in that** it contains 10-24% by weight of acetylsalicylic acid, 0,005 to 2% by weight of saccharin and/or cyclamic acid, 20-80% by weight of methoxypropanol, in particular 1-methoxy-2-propanol, and 1-30% by weight of benzyl alcohol and/or anethol as well as propylene glycol and/or diethylene glycol monoethyl ether and oleic acid and tocopherol (vitamin E).

10. A stable acetylsalicylic acid solution according to any one of claims 5 to 9 for dermal or sublingual application.

## Revendications

1. Procédé de fabrication de solutions stables d'acide acétylsalicylique avec des solvants organiques non aqueux pharmaceutiquement compatibles [tels que l'isosorbide de diméthyle, le propylèneglycol ou l'éther monoéthylique de diéthylèneglycol], **caractérisé en ce qu'**on ajoute à la solution 0,005% à 2% en poids d'un imide d'acide cyclique et/ou 0,005% à 2% en poids d'un acide sulfamique ainsi que, de préférence, 1% à 40% en poids, par rapport au poids de la solution, d'un composé de formule générale : 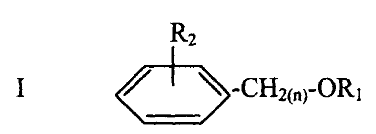 dans laquelle R₁ désigne H, CH₃ ou C₂H₅, n=0 ou 1 et R₂ désigne H ou un ou plusieurs radicaux alkyle inférieur, alkyloxy inférieur ou alcényle inférieur ayant 1 à 5 atomes de carbone.

2. Procédé selon la revendication 1, **caractérisé en ce qu'**on utilise de l'acide cyclamique comme acide sulfamique.

3. Procédé selon la revendication 1 ou 2, **caractérisé en ce qu'**on utilise un imide d'acide sulfonique ou de la saccharine comme imide d'acide cyclique.

4. Procédé selon la revendication 1, 2 ou 3, **caractérisé en ce qu'**on utilise comme composé de formule générale I de l'anéthol et/ou de l'alcool benzylique.

5. Solution stable d'acide acétylsalicylique selon l'une quelconque des revendications 1 à 4, **caractérisée en ce qu'**elle contient 5% à 15% en poids d'acide acétylsalicylique, 10% à 40% en poids de propylèneglycol, 0 à 40% en poids d'isosorbide de diméthyle, 0 à 70% en poids d'éther monoéthylique de diéthylèneglycol et 0,005% à 2% en poids de saccharine et/ou d'acide cyclamique et/ou 1% à 40% en poids d'anéthol et/ou d'alcool benzylique.

6. Solution stable selon la revendication 5, **caractérisée en ce que** la solution contient 12,5% en poids d'acide acétylsalicylique et, respectivement, 30% en poids de propylèneglycol et d'isosorbide de diméthyle, qui peuvent tous deux être remplacés totalement ou partiellement par de l'alcool benzylique ou de l'anéthol, ou éventuellement par un mélange formé des deux composants, le reste étant formé d'éther monoéthylique de diéthylèneglycol ainsi que de 0,1% à 0,5% en poids, par rapport à la solution, de saccharine et/ou d'acide cyclamique.

7. Solution stable selon la revendication 5 ou 6, **caractérisée en ce que** la solution contient 20% en poids de propylèneglycol, 65% en poids d'éther monoéthylique de diéthylèneglycol et 0,1% en poids de saccharine et/ou d'acide cyclamique et/ou 0,5% à 5% en poids d'anéthol.

8. Solution stable selon l'une quelconque des revendications 5 à 7, **caractérisée en ce que** la solution se présente sous la forme d'un atomiseur à pompe avec un obturateur de produit.

9. Solution stable selon l'une quelconque des revendications 5 à 8, **caractérisée en ce qu'**elle contient 10% à 24% en poids d'acide acétylsalicylique, 0,005% à 2% en poids de saccharine et/ou d'acide cyclamique, 20% à 80% en poids de méthoxypropanol, en particulier de 1-méthoxy-2-propanol, et 1% à 30% en poids d'alcool benzylique et/ou d'anéthol ainsi que du propylèneglycol et/ou de l'éther monoéthylique de diéthylèneglycol et de l'acide oléique et du tocophérol (vitamine E).

10. Solution stable d'acide acétylsalicylique selon l'une quelconque des revendications 5 à 9 pour application dermique ou sublinguale.
